# EUROPEAN PATENT APPLICATION

(11) **EP 4 119 071 A1**
(43) Date of publication of application: **18.01.2023**
(21) Application number: 22184572.0
(22) Date of filing: 13.07.2022
(51) Int. Cl.: A61B 17/221

(54) **CLOT RETRIEVAL DEVICE FOR REMOVING CLOT FROM A BLOOD VESSEL**

(30) Priority: 14.07.2021 US 202117375606
(71) Applicant: Neuravi Limited, Galway H91 K5YD (IE)
(72) Inventor: CASEY, Brendan, Galway, H91 K5YD (IE); VALE, David, Galway, H91 K5YD (IE)
(74) Representative: Carpmaels & Ransford LLP

(57) **Abstract**

A clot removal device that includes an expandable structure comprising interconnected struts configured to engage with clot. The interconnected struts are configured in the expanded clot engaging deployed configuration so at least a portion of the plurality of interconnected struts interpenetrates clot. The expandable structure includes a distal section, a proximal pinch section proximal of the distal section, and a clot capture mechanism.

## Description

### Field

The present disclosure generally relates to devices and methods for removing blockages from blood vessels during intravascular medical treatments.

### Background

Clot retrieval devices are used in mechanical thrombectomy for endovascular intervention, often in cases where patients are suffering from conditions such as acute ischemic stroke (AIS), myocardial infarction (MI), and pulmonary embolism (PE). Acute obstructions may include clot, misplaced devices, migrated devices, large emboli and the like. Thromboembolism occurs when part or all of a thrombus breaks away from the blood vessel wall. This clot (now called an embolus) is then carried in the direction of blood flow. An ischemic stroke may result if the clot lodges in the cerebral vasculature. A pulmonary embolism may result if the clot originates in the venous system or in the right side of the heart and lodges in a pulmonary artery or branch thereof. Clots may also develop and block vessels locally without being released in the form of an embolus-this mechanism is common in the formation of coronary blockages. There are significant challenges associated with designing clot removal devices that can deliver high levels of performance. First, there are a number of access challenges that make it difficult to deliver devices. In cases where access involves navigating the aortic arch (such as coronary or cerebral blockages) the configuration of the arch in some patients makes it difficult to position a guide catheter. These difficult arch configurations are classified as either type 2 or type 3 aortic arches with type 3 arches presenting the most difficulty.

The tortuosity challenge is even more severe in the arteries approaching the brain. For example, it is not unusual at the distal end of the internal carotid artery that the device will have to navigate a vessel segment with a 180° bend, a 90° bend and a 360° bend in quick succession over a few centimeters of vessel. In the case of pulmonary embolisms, access is through the venous system and then through the right atrium and ventricle of the heart. The right ventricular outflow tract and pulmonary arteries are delicate vessels that can easily be damaged by inflexible or high profile devices. For these reasons it is desirable that the clot retrieval device be compatible with as low profile and flexible a guide catheter as possible.

Second, the vasculature in the area in which the clot may be lodged is often fragile and delicate. For example, neurovascular vessels are more fragile than similarly sized vessels in other parts of the body and are in a soft tissue bed. Excessive tensile forces applied on these vessels could result in perforations and hemorrhage. Pulmonary vessels are larger than those of the cerebral vasculature, but are also delicate in nature, particularly those more distal vessels.

Third, the clot may comprise any of a range of morphologies and consistencies. For example, clot can be difficult to grip and improper grip can lead to fragmentation which may cause embolization. Long strands of softer clot material may also tend to lodge at bifurcations or trifurcations, resulting in multiple vessels being simultaneously occluded over significant lengths. More mature and organized clot material is likely to be less compressible than softer fresher clot, and under the action of blood pressure it may distend the compliant vessel in which it is lodged. Furthermore, the inventors have discovered that the properties of the clot may be significantly changed by the action of the devices interacting with it. In particular, compression of a blood clot causes dehydration of the clot and results in a dramatic increase in both clot stiffness and coefficient of friction.

The challenges described above need to be overcome for any devices to provide a high level of success in removing clot and restoring flow. Existing devices do not adequately address these challenges, particularly those challenges associated with vessel trauma and clot properties.

### Summary

It is an object of the present design to provide devices and methods to meet the above-stated needs. It is therefore desirable for a clot retrieval device to remove clot from cerebral arteries in patients suffering AIS, from coronary native or graft vessels in patients suffering from MI, and from pulmonary arteries in patients suffering from PE and from other peripheral arterial and venous vessels in which clot is causing an occlusion.

In some examples, a clot removal device is disclosed for removing clot from a vessel. The device can include an expandable structure including interconnected struts configured to engage with clot and including a constrained delivery configuration and an expanded clot engaging deployed configuration. The interconnected struts can be configured in the expanded clot engaging deployed configuration so at least a portion of the plurality of interconnected struts interpenetrates the clot. The expandable structure can include a distal section; and a proximal pinch section proximal of the distal section. The proximal pinch section can be configured to pinch clot on movement from the expanded clot engaging deployed configuration to the at least partially constrained clot pinching configuration. A clot capture mechanism can be included with a proximal end, a distal end, a shaft extended from the proximal end and distally extended from a proximal end of the proximal pinch section; and a capture member extended from the distal end of the shaft and within the distal section.

In some examples, the capture member can include a proximal mouth.

In some examples, the shaft can include a curve and/or pitch equivalent to the pinch section

In some examples, the capture member can terminate at or adjacent a distal end of the distal section.

In some examples, the capture member can terminate at or adjacent a proximal end of the distal section.

In some examples, the expandable structure can include an open distal end.

In some examples, the proximal pinch section can be non-cylindrical and include a diameter less than a diameter of the distal section.

In some examples, the proximal mouth can include a support frame forming a self-expandable fragment capture net coupled to the support frame.

In some examples, the proximal mouth can be formed by a proximal mouth structure including a diameter approximately equal to a diameter of the distal section. The proximal mouth structure can include a fragment protection member with closed cells smaller than closed cells of the distal and proximal pinch sections, a distalmost end of the proximal mouth structure being positioned at the distal end of the distal section.

In some examples, a proximal end of the capture member can be positioned at or adjacent where the proximal pinch section meets the distal section.

In some examples, a proximal end of the capture member can be positioned distal of where the proximal pinch section meets the distal section.

In some examples, a proximal end of the capture member can be positioned proximal of where the proximal pinch section meets the distal section.

In some examples, the proximal pinch section can be configured such that when the plurality of interconnected struts is collapsed while in a state of interpenetration with the clot, at least a portion of the proximal pinch section pinches at least a portion of the clot.

In some examples, the capture member can be axially fixed relative to the proximal pinch section and/or distal section.

In some examples, the capture member can include a closed-cell fragment protection strut structure including a porosity less than a porosity of the proximal pinch and/or distal sections.

In some examples, the distal section can include a distal barrel section flared outwardly to form a barrel shape with the open distal end, and the interconnected struts can include at least a plurality of proximally facing struts and a plurality of distally facing struts. A length of at least some of the proximally facing struts can be larger than a length of at least some of the distally facing struts thereby varying an outward radial force of the device in the expanded clot engaging deployed configuration.

In some examples, the shaft of the clot capture mechanism and the proximal pinch section can include a spiral form including a spiral pitch ranging between approximately 10-25 mm.

In some examples, a longitudinal axis can be extended through the distal section. The shaft of the clot capture mechanism and the proximal pinch section can extend around the longitudinal axis in a spiral.

In some examples, the distal barrel section includes distal radiopaque markers attached to distal most ends of at least two of the interconnected struts that form the distal barrel section.

In some examples, a plurality of radiopaque markers can be positioned at a transition between the proximal pinch section and the distal barrel section.

In some examples, the proximal pinch section can include a clot engaging element configured to exert an outward radial force when deployed within the blood vessel having an inner diameter which is lower than that of the expanded deployed configuration. The clot engaging element can be configured to exert an outward radial force varying in a generally sinusoidal pattern along a length of the clot engaging element.

In some examples, the generally sinusoidal pattern includes a wave pattern including an amplitude that decreases along a length of proximal pinch section, the amplitude can be higher at a proximal end and lower at a distal end of the clot engaging element.

In some examples, the generally sinusoidal pattern can include a wave pattern. An elongate member can also include a proximal end and a distal end, the distal end being connected to a proximal end of the expandable structure. A proximal joint can be positioned between the elongate member and the expandable structure, the proximal joint including a step at the distal end of the elongate member.

In some examples, the distal section included an inner expandable body with a framework of struts. An outer expandable body can be included with a framework of struts forming an outer body radially surrounding the inner body during the collapsed delivery configuration and the expanded deployed configuration. The outer expandable body can be expandable to a radial extent to define a clot reception space.

In some examples, the framework of struts of the outer expandable body can include a plurality of discontinuous expandable members spaced from adjacent expandable members, struts of each expandable forming closed cells with at least some struts terminating in radially separated distal apexes free from connection to an adjacent closed cell.

In some examples, the inner expandable body can include a porous inner body flow channel.

In some examples, the inner expandable body can include a first scaffolding segment including a plurality of the closed cells, a second scaffolding segment with a plurality of the closed cells and located distally of the first scaffolding segment. The inner body can extend inside the first and second scaffolding segments. A clot inlet mouth can be located between the first scaffolding segment and the second scaffolding segment for receiving the clot or fragments thereof.

In some examples, the clot inlet mouth can be unscaffolded and include an opening larger than any one of the plurality of cells of the first and second scaffolding segments .

In some examples, the clot inlet mouth can be disconnected or otherwise form a disconnection between the first scaffolding segment and the second scaffolding segment over a circumferential arc of approximately 180 degrees.

In some examples, the device can include an open distal end.

In some examples, the proximal pinch section can include a diameter more than a diameter of the distal section.

In some examples, the proximal pinch section can be substantially planar, straight, or otherwise elongated.

In some examples, the proximal pinch section can be cylindrical.

In some examples, proximal struts of the outer expandable body can pass through one or more of the cells of the proximal pinch section.

In some examples, proximal struts of the outer expandable body are positioned both inside and outside of the proximal pinch section.

In some examples, the distal section can include an inner expandable body including a porous inner body flow channel.

In some examples, the capture member can include an outer expandable body including a framework of struts forming an outer body radially surrounding the distal section during the collapsed delivery configuration and the expanded deployed configuration, wherein the outer expandable body is expandable to a radial extent to define a clot reception space between it and the distal section.

In some examples, the outer expandable body can include a closed distal end and at least one scaffolding segment proximal of the closed distal end. The at least one scaffolding segment can include a plurality of the closed cells, the distal section extending inside the least one scaffolding segment.

In some examples, the framework of struts of the outer expandable body can be a plurality of discontinuous expandable members forming closed cells with at least some struts terminating in radially separated distal apexes free from connection to an adjacent closed cell.

In some examples, the closed end can be formed by a distal scaffolding zone, including a plurality of struts that distally taper with closed cells smaller than cells proximal thereof in the outer expandable body.

In some examples, the plurality of closed cells of the distal scaffolding zone can include a protective strut structure.

In some examples, the distal section including an inner expandable body including a porous inner body flow channel terminating in a plurality of distal struts each having a first end coupled to a distal end of the porous inner body flow channel and a second end coupled to each other so as to form a connection point.

In some examples, the plurality of distal struts is spiraled.

In some examples, the plurality of distal struts is configured in a bulged or flared pattern.

In some examples, the distal section includes an inner expandable body including a porous inner body flow channel terminating in one or more distal struts to define a protective strut structure, configured to prevent distal egress of clot or clot fragments from the device. The protective strut structure can include a diameter larger than a diameter of the porous inner body flow channel and being positioned at or adjacent the closed distal end of the capture member.

In some examples, a method of capturing clot is disclosed. The method can include deploying the expandable structure of any device of this disclosure into contact with at least a portion of the clot; pinching at least a portion of the clot with the proximal pinch section; and capturing, by the clot capture mechanism, one or more fragments of the clot.

Other aspects and features of the present disclosure will become apparent to those of ordinary skill in the art, upon reviewing the following detailed description in conjunction with the accompanying figures.

### Brief Description of the Drawings

The above and further aspects of this disclosure are further discussed with the following description of the accompanying drawings, in which like numerals indicate like structural elements and features in various figures. The drawings are not necessarily to scale, emphasis instead being placed upon illustrating principles of the disclosure. The figures depict one or more implementations of the inventive devices, by way of example only, not by way of limitation. It is expected that those of skill in the art can conceive of and combining elements from multiple figures to better suit the needs of the user.
Fig. 1 illustrates an isometric view of a device according to this disclosure.
Fig. 2A is an isometric view of a fragment protection member according to this disclosure.
Fig. 2B is an isometric view of a pinch member according to this disclosure.
Fig. 3 illustrates an isometric view of a device according to this disclosure.
Fig. 4A is an isometric view of a fragment protection member according to this disclosure.
Fig. 4B is an isometric view of a pinch member according to this disclosure.
Fig. 5 is an isometric view of a device according to this disclosure.
Fig. 6A is an isometric view of a pinch member according to this disclosure.
Fig. 6B is an isometric view of an outer member according to this disclosure.
Fig. 7A is an isometric view of a device according to this disclosure.
Fig. 7B is an isometric view of a device according to this disclosure.
Fig. 8A shows one step in a method of use of a clot retrieval device of this disclosure.
Fig. 8B shows one step in a method of use of a clot retrieval device of this disclosure.
Fig. 9A shows one step in a method of use of a clot retrieval device of this disclosure.
Fig. 9B shows one step in a method of use of a clot retrieval device of this disclosure.
Fig. 10 shows one step in a method of use of a clot retrieval device of this disclosure.
Fig. 11 is a flow diagram illustrating a method of removing a clot from a blood vessel of a patient, according to aspects of the present disclosure.

### Detailed Description

Specific examples of the present disclosure are now described in detail with reference to the Figures, where identical reference numbers indicate elements which are functionally similar or identical. The examples address many of the deficiencies associated with traditional catheters, such as inefficient clot removal and inaccurate deployment of catheters to a target site.

Accessing the various vessels within the vascular, whether they are coronary, pulmonary, or cerebral, involves well-known procedural steps and the use of a number of conventional, commercially-available accessory products. These products, such as angiographic materials and guidewires are widely used in laboratory and medical procedures. When these products are employed in conjunction with the system and methods of this disclosure in the description below, their function and exact constitution are not described in detail.

The following detailed description is merely exemplary in nature and is not intended to limit the disclosure or the application and uses of the disclosure. Although the description of the disclosure is in many cases in the context of treatment of intracranial arteries, the disclosure may also be used in other body passageways as previously described.

It will be apparent from the foregoing description that, while particular embodiments of the present disclosure have been illustrated and described, various modifications can be made without departing from the spirit and scope of the disclosure. For example, while the embodiments described herein refer to particular features, the disclosure includes embodiments having different combinations of features. The disclosure also includes embodiments that do not include all of the specific features described. Specific embodiments of the present disclosure are now described in detail with reference to the figures, wherein identical reference numbers indicate identical or functionality similar elements. The terms "distal" or "proximal" are used in the following description with respect to a position or direction relative to the treating physician. "Distal" or "distally" are a position distant from or in a direction away from the physician. "Proximal" or "proximally" or "proximate" are a position near or in a direction toward the physician.

Accessing cerebral, coronary and pulmonary vessels involves the use of a number of commercially available products and conventional procedural steps. Access products such as guidewires, guide catheters, angiographic catheters and microcatheters are described elsewhere and are regularly used in catheter lab procedures. It is assumed in the descriptions below that these products and methods are employed in conjunction with the device and methods of this disclosure and do not need to be described in detail.

The following detailed description is merely exemplary in nature and is not intended to limit the disclosure or the application and uses of the disclosure. Although the description of the disclosure is in many cases in the context of treatment of intracranial arteries, the disclosure may also be used in other body passageways as previously described.

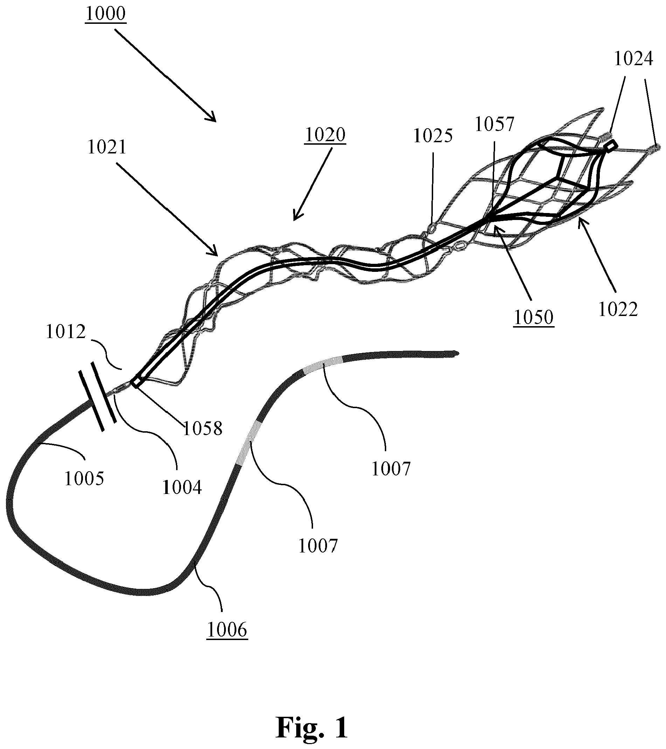

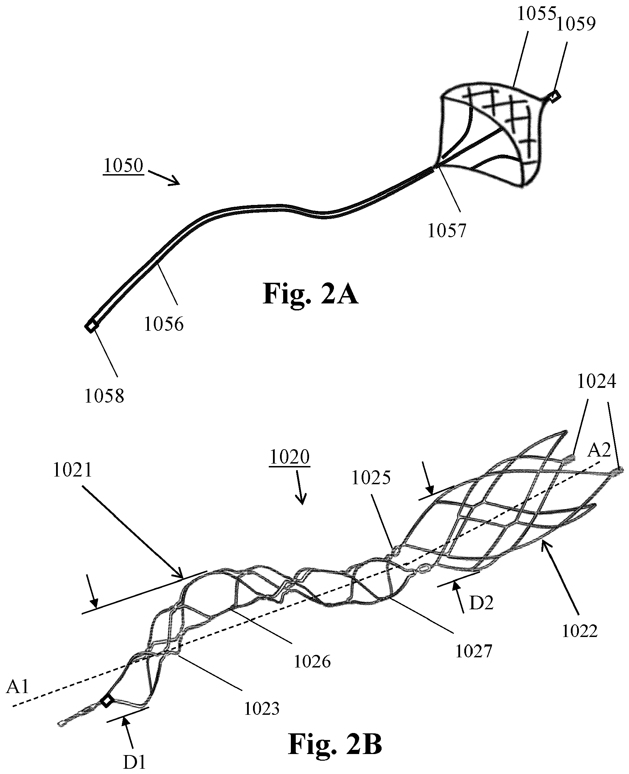

A common theme across many of the disclosed designs is a multi-layer construction in which the device in certain instances can include a proximal pinch member which, at times, can include a fragment protection member and/or other elements to facilitate clot capture, whereby such members can be directly or indirectly connected to an elongate shaft. Turning to Fig. 1, one example device 1000 according to this disclosure is illustrated. Member 1020 includes a proximal pinch section 1021 and a distal barrel section 1022. Device 1000 can include a fragment protection member 1050, as discussed more particularly in Fig. 2A, that is positioned inside section 1022, which can improve the efficacy of the fragment protection.

Distal radiopaque markers 1025 can be positioned between sections 1021, 1022 and can be included in one or more distal crowns 1024. Crowns 1024 can be disconnected distal apexes. In this case the proximal pinch section 1021 can be heat set into a spiral shape (e.g., a spiral about axis A1 shown in Fig. 2B). The spiral may include a spiral pitch with approximately 14 mm (within a range of approximately 10-25 mm); spiral outer diameter (e.g., diameter D1 of Fig. 2B) of approximately 5 mm (within a range of 4.0 to 10 mm); and the spiral typically may form an approximately 360° curve, or range from approximately 180 to 720°. Diameter D2 of section 1022 can be smaller than diameter D1 of section 1021. However, device 1000 is not so limited and instead diameter D2 can be equivalent to and/or larger than diameter D1, as needed or required

Pinch section 1021 can include a ring of cells 1023, which can include of circumferential cells. The number of cells in the circumferential ring can vary from 2 to 5, but in the preferred embodiment is 3 or 4 cells. Portions 1026 of the device 1000 between the cells 1023, (e.g., the cells that are embedding) can have low radial force and a low level of scaffolding. The low level of scaffolding is achieved by minimizing the potential surface contact area between the device struts and the clot in this area 1026. In this embodiment the connecting struts 1027 can be curved towards the center-line at the mid-point to further reduce the strut contact force and area with the clot. This low surface contact area and radial force allows the clot to protrude into this section of the device 1000 when it is deployed in an occlusive clot. Partial re-sheathing of the device 1000 with a microcatheter or intermediate catheter can then pinch this protruding clot between the tip of the catheter and the proximal struts 1026 of the embedding ring of cells 1023.

A longitudinal axis A2 of the distal barrel section 1022 may be offset from the spiral to assist in achieving uniform (low strain) connection between the sections. In this device 1020, the distal end of the spiral section can be orientated so that it is perpendicular to the proximal face of the barrel section 1022. In this orientation, both the struts connecting the spiral section to the barrel section 1022 are equal length and have equivalent levels of strain regardless of the cut pattern orientation on the heat forming mandrel. In other iterations the spiral section may be orientated at an angle to the barrel section 1022.

Section 1021 can include cells, struts, and a variety of shapes and designs configured for pinching fibrin rich clots, including those described in U.S. Pat. No. 10,292,723; U.S. Pat. No. 10,363,054; U.S. App. No. 15/359,943; U.S. App. No. 16/021,505; and U.S. App. No. 16/330,703, each of which are incorporated by reference in their entirety as if set forth verbatim herein. Compression of the clot by member 1020, including by section 1021, can alter the clot properties and make the clot less amenable to retrieval by making it firmer and "stickier" as described in WO2012/120490A, the entire contents of which are herein incorporated by reference.

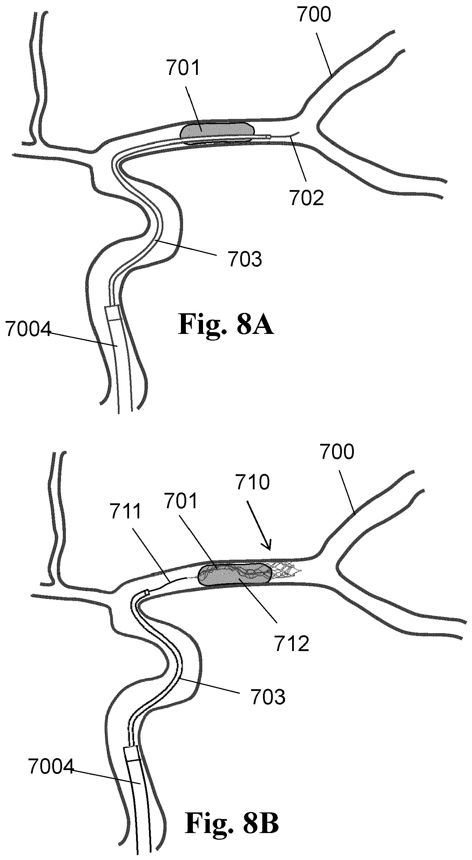

In some examples, distal advancement of the microcatheter relative to the device 1000 will can compress the clot between the catheter tip and the struts of the device 1000, including but not limited to section 1021 increasing the pinch on the clot (Fig. 2c) and the security of the trapped clot segment. The user may feel this pinch as a resistance and stop advancing the microcatheter, or alternatively the user may advance the microcatheter a fixed distance over the device 1000 (for example 30% to 50% of the device length) before retracting the device 1000 and microcatheter together. This process is shown more clearly in Figs. 8A - 10.

The relative tension between the device 1000 and the microcatheter needs to be maintained to ensure the pinch between the device 1000 and clot does not deteriorate. By retracting the device 1000 and the microcatheter together, the occlusive clot can be dislodged and retracted back into the access guide catheter or introducer sheath and be removed from the patient. This disclosure is particularly suited to the dislodgement and retraction of clots which have a high fibrin content (typically higher than 40% fibrin content) and other clots which are difficult to dislodge and retrieve with known stent retriever designs and currently may require multiple passes to remove the clot from the vasculature. This disclosure may also create a clot pinch by advancing an intermediate catheter in the same manner as described here for the microcatheter.

The device 1000 of this disclosure is intended to facilitate clot retrieval by expanding between the clot and the vessel wall in such a way as to engage with the clot over a surface area and do so with minimal compression of the clot. In some examples, the pinch can be achieved by forwarding a microcatheter or intermediate catheter over the device until a portion of clot is compressed between the catheter and member 1020. However, actuation of member 1020 is not so limited and can be also carried out by pulling one or more pull members attached therewith, by delivering a current to one or more of strut members of member 1020 to cause to change from a collapsed configuration to pinch configuration, and/or the like. This pinch facilitates removal of the clot as it increases the grip of the device 1000 on the clot, particularly fibrin rich clots. It may also elongate the clot reducing the dislodgement force by pulling the clot away from the vessel wall during the dislodgement process.

Device 1000 can have an elongate shaft 1006 having a distal end that extends interior of the artery and a proximal end that extends exterior of the artery. Shaft 1006 may be a tapered wire shaft, and may be made of stainless steel, MP35N, Nitinol or other material of a suitably high modulus and tensile strength. Shaft 1006 may have indicator bands 1007 to indicate when the distal end of device 1000 is approaching the end of the microcatheter during insertion. Shaft 1006 can have a coil 1004 adjacent its distal end. Coil 1004 may be metallic and may be formed from stainless steel or from a more radiopaque material such as platinum or gold for example or an alloy of such a material. In other examples, coil 1004 can be coated with a low friction material or have a polymeric jacket positioned on the outer surface of the coil 1004.

Adjacent to coil 1004 a sleeve 1005 may be positioned on shaft 1006. Sleeve 1005 may be polymeric and may be positioned over a tapered section of shaft 1006. Sleeve 1005 may be rendered radiopaque through the addition of a filler material such as tungsten or barium sulphate. However, other radiopaque materials are contemplated, including but not limited to Bismuth SubCarbonate, Barium OxyChloride, Gold, Platinum, Iridium, Tantalum or an alloy of any of these materials. The sleeve 1005 and shaft 1006 may be coated with a material to reduce friction and thrombogenicity. The coating may include a polymer, a low friction lubricant such as silicon, a hydrophilic or a hydrophobic coating. This coating may also be applied to device 1000.

Turning to Fig. 2A is an isometric view of fragment protection member 1050 without being positioned with member 1020 according to this disclosure. Similarly, Fig. 2B is an isometric view of member 1020 according to this disclosure without member 1050 positioned therewith. Member 1050 can include of a wire 1056 connecting fragment protection member 1050 with a proximal joint 1058. The leading edge 1057 of the fragment protection structure 1055, as shown in Fig. 1, can be located inside the barrel section 1022 and aligned with and/or substantially adjacent distal crowns 1024. In one example, a distal end 1059 of member 1050 can be coextensive with crowns 1024. Member 1050 may also be fixedly positioned within the barrel section 1022 or may be movable between one or more predetermined positions, as needed or required. Member 1050 being positioned as shown and described is particularly advantageous for capturing fragments of clot by its smaller, mesh sized cells of structure 1055 without impacting the pinch capability of pinch section 1021. Structure 1055 and corresponding elements shown in Fig. 2A can be attached to wire 1056, which can be a shaft, with the same or similar spiral curvature and pitch as the pinch section 1021. As shown, member 1050 include a variety of net or basket designs to contain any fragments that might be released from the main body of clot. Such anti-fragmentation features of member 1050 are also disclosed that sit at the distal end of device 1000, which minimize the risk of distal embolization during the clot removal procedure.

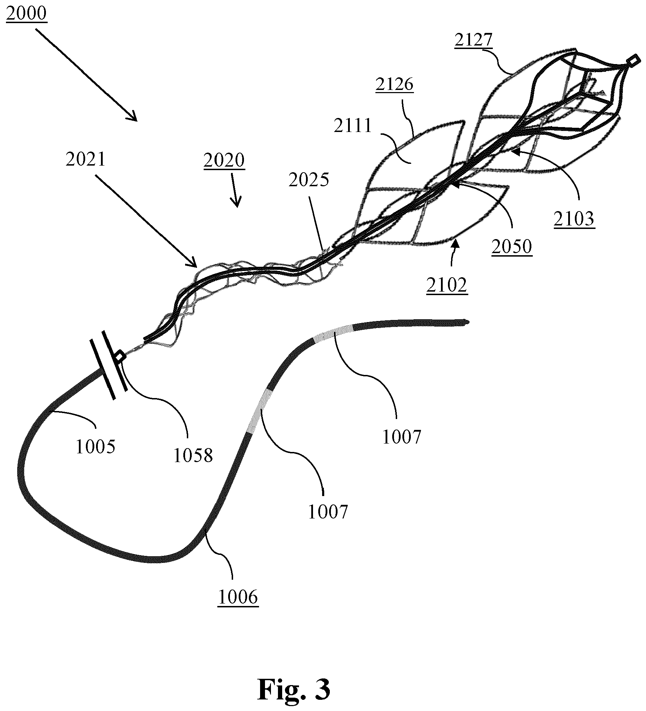

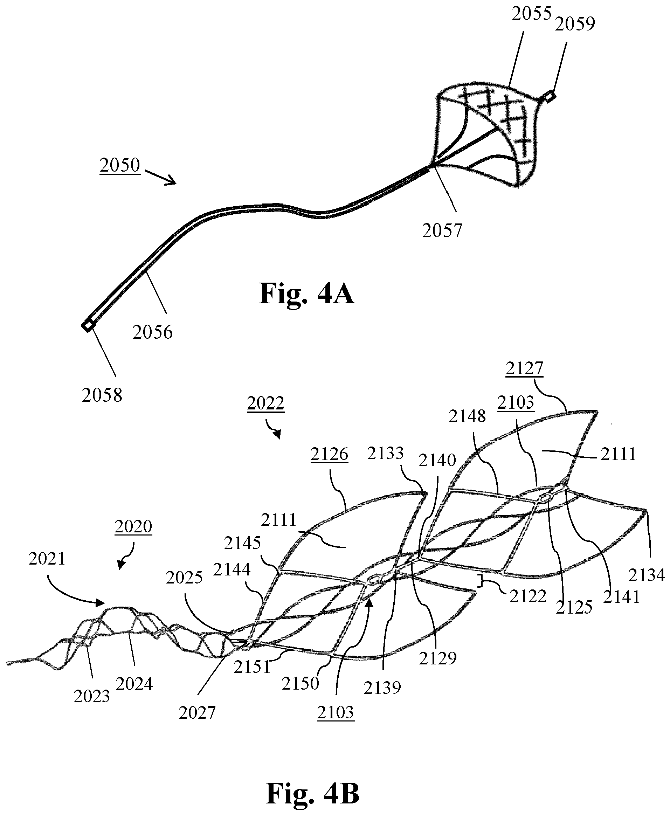

Referring to FIG. 3 there is illustrated another device 2000 according to the disclosure. Similar to device 1000, device 2000 can be attached to shaft 1006 and include a pinch member 2020, a distal section 2022 distally extended from member 2020, and a fragment protection member 2050 positioned internal to section 2022. Distal section 2022 can be understood as incorporating some or all features of the clot retrieval device described in U.S. Pat. No. 8,777,976; 8,852,205; 9,402,707; 9,445,829; 9,642,639; 10,292,722; 10,299,811; 10,588,649; and 10,610,246, each of which are incorporated by reference in their entirety as if set forth verbatim herein. Pinch member 2020 can include a proximal pinch section 2021, largely the same as pinch section 1021. Fragment protection member 2050 is more clearly shown in Fig. 4A while member 2020 is more clearly shown in Fig. 4B.

In Fig. 4A, member 2050 can include of a wire 2056, similar to wire 1056, connecting fragment protection member 2050 with a proximal joint 2058. The leading edge 2057 of the fragment protection structure 2055, as shown in Fig. 3, can be located inside section 2022 (e.g., member 2102) and aligned with and/or substantially adjacent distal crowns of section 2022. In one example, a distal end 2059 of member 2050 can be coextensive with crowns 2134 of section 2022. Member 2050 may also be fixedly positioned within section 2022 or may be movable between one or more predetermined positions, as needed or required. Member 2050 being positioned as shown and described is particularly advantageous for capturing fragments of clot by its smaller, mesh sized cells of structure 2055 without impacting the pinch capability of pinch section 2021 and/or clot capture by section 2022. Structure 2055 and corresponding elements shown in Fig. 4A can be attached to wire 2056, which as with wire 1056, can be a shaft, with the same or similar spiral curvature and pitch as the pinch section 2021.

Distal section 2022 can distally extend from connecting struts 2027 and can include an outer expandable member 2102 and an inner expandable member 2103 to facilitate restoration of blood flow through clot immediately after device 2100 is deployed at an obstructive site. Member 2103 is configured to self-expand upon release from a restraining sheath (e.g., a microcatheter) to a diameter larger than that of member 2102. Members 2102 and 2103 can specifically have a collapsed configuration for delivery and an expanded configuration for flow restoration and fragmentation protection. Members 2102, 2103 can be joined at the proximal and/or distal ends during assembly to minimize tension within members 2102, 2103 during use. In other examples, member 2103 may not be connected to the distal end of member 2103 at all or may be constrained within member 2102 without being fixedly attached.

Expansion of member 2102 can cause compression and/or displacement of the clot during expansion. When an expandable body provides a high level of scaffolding, the clot is compressed. When an expandable body provides an escape path or opening the expanding body will urge the clot towards the opening. However, if the expandable body provides only modest scaffolding the clot will be displaced but since the clot has many degrees of freedom it may move in a variety of different directions and therefore cannot be controlled. By providing a tubular expandable body where the length of the tubular expandable body is substantially as long as the length of the occlusive clot or longer, many of the degrees of movement freedom available to the clot are removed.

As shown, member 2102 can include two (2) expandable members of member 2102. However, any number of expandable members are contemplated for use with section 2022. Members 2102 and 2103 have a collapsed configuration for delivery and an expanded configuration for clot retrieval, flow restoration and fragmentation protection in connection with member 2050. In some examples, member 2103 can have a generally tubular body section. In other examples, member 2103 can have a non-cylindrical cross-section, may be non-uniform in diameter, and may have tailored strut patterns to provide regions of differing radial force or flexibility. Member 2103 can be configured so as to provide a flow lumen or flow channel (e.g., generally cylindrical section) through distal section 2022 to facilitate restoration of blood flow past the clot upon deployment. In one embodiment, member 2103 is configured to scaffold the flow channel through the clot to prevent the liberation of fragments which might otherwise lodge in the distal vasculature. The length of member 2102 can be substantially the same as the length of member 2103 in the freely expanded configuration and the loaded, collapsed configuration. Members 2102 and 2103 are preferably made of a superelastic or pseudo-elastic material such as Nitinol or another such alloy with a high recoverable strain.

Inlet openings 2122 are provided in member 2102 whereby inlets 2122 can provide a primary movement freedom available to the clot and so the expansion of member 2102 urges the clot into reception space 2111. Member 2102 can have multiple inlet mouths 2122 to accept clot. Inlet mouths 2122 can be configured to allow portions of the clot to enter reception space 2111 and thus allow the clot to be retrieved without being excessively compressed. This is advantageous because the inventors have discovered that compression of clot causes it to dehydrate, which in turn increases the frictional properties of the clot, and increases its stiffness, all of which makes the clot more difficult to disengage and remove from the vessel. This compression can be avoided if the clot migrates inward through the wall of member 2102 as the porous structure migrates outward towards the vessel wall.

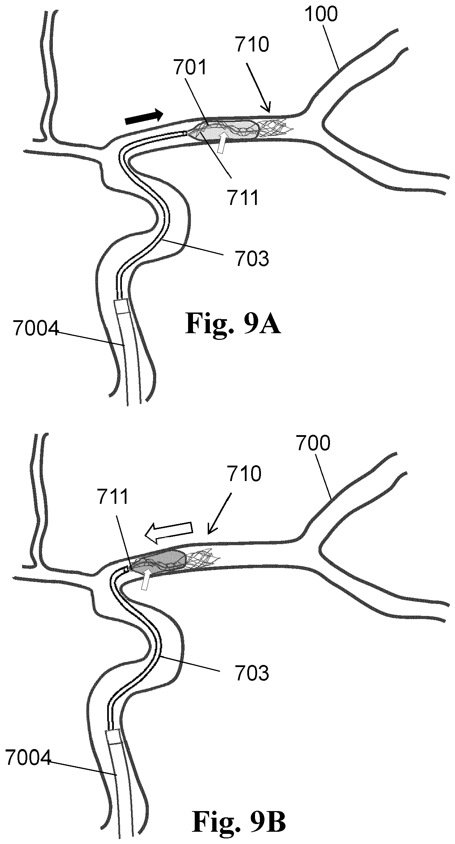

The inlet mouths 2122 can also provide the added benefit of allowing member 2102 when retracted to apply a force to the clot in a direction substantially parallel to the direction in which the clot is to be pulled from the vessel (i.e. substantially parallel to the central axis of the vessel). This means that the outward radial force (e.g., as denoted by arrows in Figs. 9A and 9B) applied to the vasculature may be kept to a minimum, which in turn means that the action of the clot retrieval distal section 2022 on the clot does not serve to increase the force required to dislodge the clot from the vessel, thus protecting delicate cerebral vessels from harmful radial and tensile forces.

Member 2102, as shown, can include proximal struts (e.g., struts 22144, 2155) connected at their proximal ends to section 2021, including at struts 2027 positioned on or adjacent a distal end of section 2021, to a first expandable member 2126. As shown, struts 22144, 2155 may have a tapered profile to ensure a gradual stiffness transition from section 2021 to the clot engagement section of the distal section 2022. Member 2126 can be connected to a second expandable member 2127 by a plurality of connecting arms 2129, which can run from a proximal junction 2139 to a distal junction 2140. Arms 2129 can include generally straight struts running parallel to the central axis of the device. In other embodiments these connecting arms may include a plurality of struts configured in one or more cells or may include curved or spiral arms. The region between the first and second expandable member includes two inlet mouths 2122 through which clot may pass and enter the reception space 2111 defined by the region between the inner and outer members. The segmented and hinged design of struts between member 2126 and member 2127 is also specifically tailored to achieving apposition in bends in the vasculature. The only connecting members between members 2126, 2127 can be arms 2129 which function as hinge elements configured to self-align to the neutral axis and allow the device to easily articulate in the bend.

Member 2127 can in turn be connected to a third expandable member, or any number of expandable members distally connected thereto. In some examples, member 2126 can include interconnected struts, such as those terminating in crowns 2133, 2134 with no distal connecting elements, and other struts such as 2144 terminating injunction points, such as crown 2145. Struts in the expandable members may be configured so that during loading, multiple crowns (e.g., crowns 2145, 2150) do not align at the same distance. During loading or resheathing, a higher force can be generally required to load a crown than a strut into the sheath. Accordingly, if multiple crowns are loaded at the same time the user may notice an increase in loading force. Similarly, second expandable member 2127 can include interconnected struts terminating in crowns 2134 with no distal connecting elements, and other struts terminating injunction points.

In some examples, expandable members of member 2102 may include one or more markers 2125 with radiopaque materials such as, but not limited to, a radiodense material such as Gold, Tungsten, Tantalum, Platinum or alloy containing these or other high atomic number elements. Polymer materials (e.g., polyurethane, pebax, nylon, polyethylene, or the like) might also be employed, containing a Radiopaque filler such as Barium Sulphate, Bismuth SubCarbonate, Barium OxyChloride, Gold, Tungsten, Platinum, Iridium, Tantalum, an alloy of these materials, and/or an adhesive filled with radiopaque filler. In this respect, marker 2125 can be included as an eyelet on struts throughout member 2102. Marker 2125 can be positioned to indicate to the user the distal end of member 2102 to aid in accuracy of deployment.

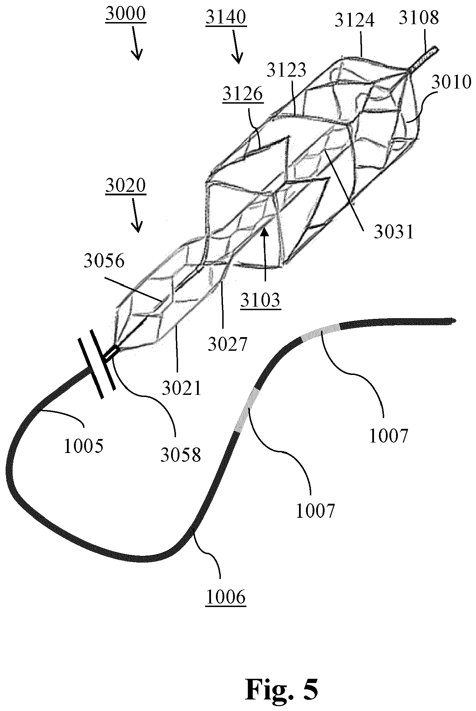

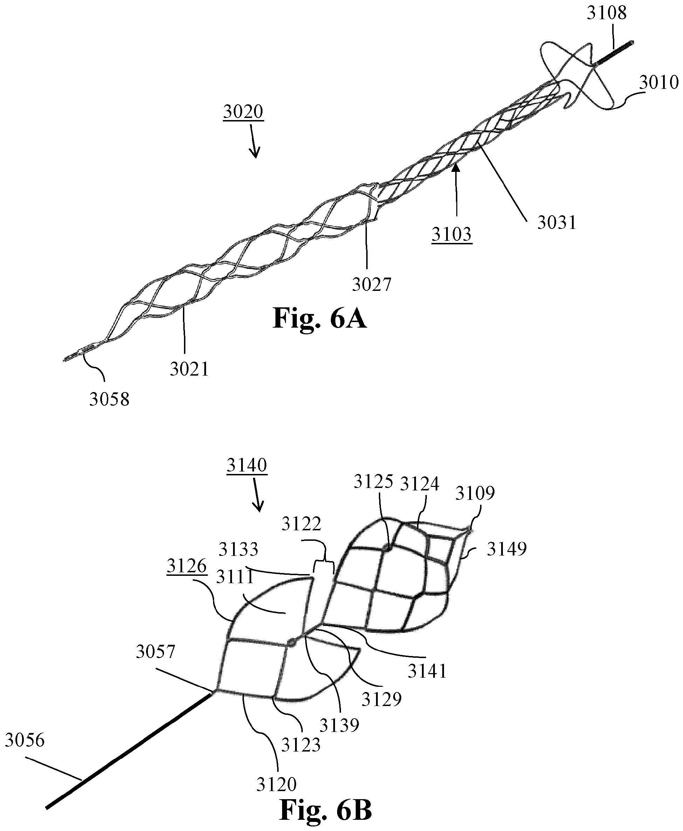

Referring to FIG. 5 there is illustrated another device 3000 according to the disclosure. Similar to devices 1000 and 2000, device 3000 can be attached to shaft 1006 and include a pinch member 3020 and a distal, inner member 3103 distally extended from member 3020. Member 3020 can be shaped with a relatively cylindrical pinch section 3021. However, member 3020 is not so limited and may instead can be non-planar, similar to member 2020, but instead of being spiraled or curved, member 3020 instead can be shaped with a relatively straight pinch section 3021. In some aspects, member 3020 can be stepped down, for example beginning at junction 3027, in diameter as it distally extends to member 3103 from pinch section 3021. Pinch section 3021 can include a plurality of diamond-shaped closed cells larger than closed cells of member 3103. Device 3000 can include expandable member 3140 extended from shaft 1006 via shaft 3056 within and/or along member 3020, as discussed more particularly in Figs. 6A-6B.

As shown more clearly in Fig. 6A, member 3020 can include a proximal pinch section 3021 that can transition to inner member 3103 that includes a series of interconnected struts that tapers from member 3020 to a narrower width at member 3103. Member 3103 can be non-planar shaped, such as flat or spiral shaped. For example, 3103 can include a flat or single cell wave shape. In another example, member 3103 can form a tubular inner flow channel, similar to member 2103. A distal end of member 3103 can include an expansile section formed of expanded struts 3010 that have a diameter greater than portions of member 3103 proximal thereof. Struts 3010 can be connected to a coil section 3108, whereby struts 3010 can provide a significant restriction to the path of a clot fragment therethrough. In some examples, coil section 3108 can include a castellated profile joined to a distal collar of the outer member 3140 during assembly.

Turning to Fig. 6B, an isometric view of member 3140 is shown. Member 3140, as shown, can include proximal shaft 3056 extended distally to junction 3057. Junction 3057 can be a joint that is offset from the inner channel 3103 (e.g., at junction 3027) so that outer struts of member 3140 do not block clot embedding in the pinch section 3021, maintaining the pinch capability. Extended therefrom can be one or more proximal struts 3120 connected at their proximal ends and at their distal ends to an expandable member 3126, which can be more clearly seen and described in U.S. App. No. 16/946,467, which is incorporated by reference in its entirety as if set forth verbatim herein. As shown, struts 3120 may have a tapered profile to ensure a gradual stiffness transition from shaft 3156 to the clot engagement section of the member 3140. Although not shown, member 3126 can be connected to a second expandable member until the distal end of member 3140.

Member 3126 can be connected to the distal section of member 3140 by a plurality of connecting arms 3129, which can run from a proximal junction 3139 to a distal junction 3141. Arms 3129 can include generally straight struts running parallel to the central axis of the member 3140. In other embodiments, connecting arms 3129 may include a plurality of struts configured in one or more cells or may include curved or spiral arms. The region between member 3126 and distal end 3109 can include at least two inlet mouths 3122 through which clot may pass and enter a reception space defined by the region between the member 3140 and member 3103, shown in member 3020 of Fig. 6A. In some examples, member 3126 can include interconnected struts terminating in crowns 3133 with no distal connecting elements, and other struts such as strut 3122 terminating in junction points 3123. The segmented and hinged design of struts between member 3126 and the distal scaffolding section distal thereof, similar to members 2126, 2127, is also specifically tailored to achieving apposition in bends in the vasculature whereby arms 3129 which function as hinge elements configured to self-align to the neutral axis and allow the device to easily articulate in the bend.

Clot inlet openings 3122 can provide a primary movement freedom available to the clot and so the expansion of member 3140 urges the clot into reception space. Member 3140 can be configured to allow portions of the clot to enter the reception space and thus allow the clot to be retrieved without being excessively compressed. This is advantageous because the inventors have discovered that compression of clot causes it to dehydrate, which in turn increases the frictional properties of the clot, and increases its stiffness, all of which makes the clot more difficult to disengage and remove from the vessel. This compression can be avoided if the clot migrates inward through the wall of member 3140 as the porous structure migrates outward towards the vessel wall.

In some examples, member 3140 may include one or more markers 3125 with radiopaque materials such as, but not limited to, a radiodense material such as Gold, Tungsten, Tantalum, Platinum or alloy containing these or other high atomic number elements. Polymer materials (e.g., polyurethane, pebax, nylon, polyethylene, or the like) might also be employed, containing a Radiopaque filler such as Barium Sulphate, Bismuth SubCarbonate, Barium OxyChloride, Gold, Tungsten, Platinum, Iridium, Tantalum, an alloy of these materials, and/or an adhesive filled with radiopaque filler. In this respect, marker 3125 can be included as an eyelet on struts throughout member 3140. Marker 3125 can be positioned to indicate to the user the distal end of member 3140 to aid in accuracy of deployment. The distal end of member 3140 can include a circumferential ring of struts connected to a series of struts 3124 that can terminate at a distal junction point 3109, which can include a collar. Struts 3124 can form a plurality of closed cells for capturing clot fragments. In some examples, member 3140 can terminate in a closed distal end while in other aspects, the distal end of member 102 can be opened or not necessarily closed. In some examples, struts 3124 may include a generally conical shape, as shown. In some examples, struts 3124 can be arranged in a generally flat plane which may be inclined or may be normal to the longitudinal axis of device 3000. Struts 3124 and 3149 can be tapered to a narrower width than those of the more proximal struts including the body of the expandable member 3126 thus creating a gradual transition in the stiffness of the device both in the expanded and collapsed states.

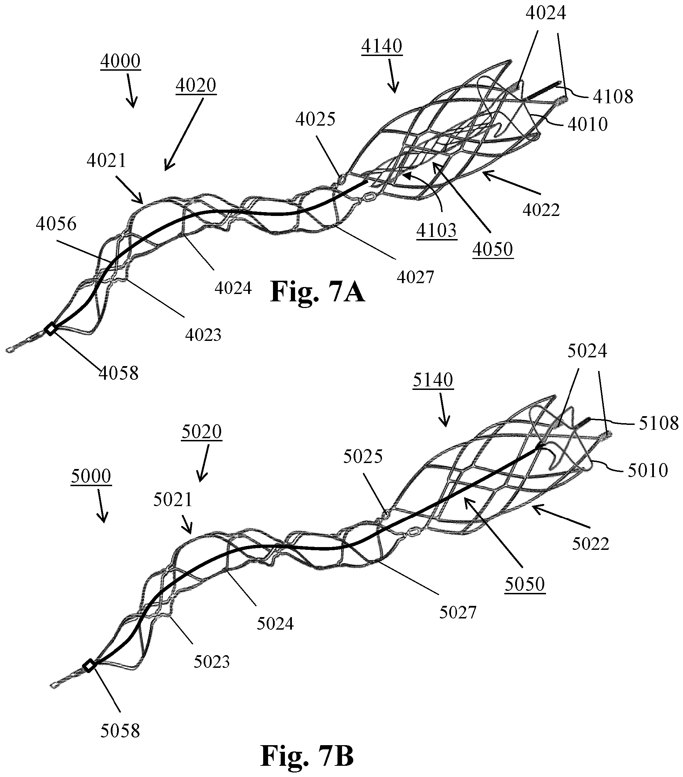

Referring to FIG. 7A there is illustrated another device 4000 according to the disclosure. Similar to devices 1000, 2000, 3000, device 4000 can be attached to shaft 4006 and include a pinch member 4020, which is structurally similar to pinch member 1020. Device 4000 can include fragment protection member 4050 with wire 4056, similar to wire 1056, connecting fragment protection member 4050 with a proximal joint 4058. Wire 4056 can distally transition to inner member 4103, which can include a tubular inner flow channel, similar to member 2103. A distal end of member 4103 can include an expansile section formed of expanded struts 4010 that have a diameter greater than portions of member 4103 proximal thereof. Struts 4010 can be connected to a coil section 4108, whereby struts 4010 can provide a significant restriction to the path of a clot fragment therethrough. In some examples, coil section 4108 can include a castellated profile joined to a distal collar of the outer member 4140 during assembly.

Referring to FIG. 7B there is illustrated another device 5000 according to the disclosure, which is similar to device 4000. Fragment protection member 5050, however, does not include any inner tubular member (e.g., such as member 4103 of device 4000). Instead, wire 5056 of member 5050 can distally transition directly to expanded struts 4010.

FIGS. 8A-10 show a method of use of a device of this disclosure. Fig. 8A shows guidewire 702 and microcatheter 703 are inserted in the vasculature 700 and are advanced across the obstructive clot 701 using conventionally known techniques. When the microcatheter 703 is positioned distal to the occlusive clot 701, the guidewire 702 is removed from the vasculature 700 to allow the clot retrieval device 710 be advanced through the microcatheter 703. It is understood the device 710 can be any of the herein disclosed clot retrieval devices and the device depicted here relates to device 1000 strictly for illustrative purposes. The device 710 is advanced in a collapsed configuration until the distal tip of the device reaches the distal end of the microcatheter 703. The microcatheter is retracted while the position of device 710 is maintained to deploy the clot retrieval device across the clot 701 in a manner that the distal end of the device is preferably positioned distal of the clot 701 (Fig. 8B). The device 710 can include a clot engagement portion 712, which here relates to pinch sections of this disclosure, such as section 1021, connected to an elongated proximal shaft portion 711.

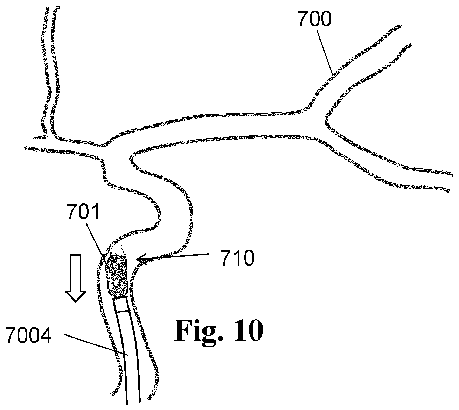

The device 710 expands so that it engages with the occlusive clot at the proximal end or along its length. The device has segments that have low levels of scaffolding and do not compress the clot but allow the clot to protrude into these low radial force areas. The device 710 may be allowed to incubate for a period of time within the clot 701 if desired, as shown in Fig. 9A. As also shown in Fig. 9A, device 710 can apply an outward radial force as denoted by the example force arrows in both Figs. 9A and 9B. Prior to retracting the device 710, the microcatheter 7103 can be forwarded distally to pinch a portion of the clot between the tip of the microcatheter and the struts and crowns of the device adjacent to the low radial force area, as shown in Fig. 9B. This pinch provides additional grip and control of the proximal end of the clot during dislodgement and retention back to the access guide catheter 7004 or introducer sheath, as shown in FIG. 10. The relative tension between the device 710 and the microcatheter 703 is maintained by the user during dislodgment and retraction to ensure the pinch on the clot is maintained. While the use of a microcatheter or intermediate catheter to pinch the clot is described as giving additional benefits when used with this invention, all the embodiments described herein can also be used to dislodge and retrieve clots without the use of catheter pinching if required.

Flow arrest in the vessel may be utilised by inflating a balloon (not shown) on the guide catheter 7004 as per standard technique. FIG. 10 illustrates the clot engaged with the device during retrieval into the guide catheter 7004. Flow occlusion, aspiration and other standard techniques may be used during the clot retrieval process. The device 710 may be rinsed in saline and gently cleaned before reloading in the insertion tool. The device 710 may be reintroduced into the microcatheter 703 to be redeployed in additional segments of occlusive clot, if required.

A range of designs are envisaged for each of these elements as described throughout this document, and it is intended that any of these elements could be used in conjunction with any other element, although to avoid repetition they are not shown in every possible combination.

Any of the heretofore clot retrieval devices and corresponding features are desirably made from a material capable of recovering its shape automatically once released from a highly strained delivery configuration. A superelastic material such as Nitinol or an alloy of similar properties is particularly suitable. The material could be in many forms such as wire or strip or sheet or tube. A particularly suitable manufacturing process is to laser cut a Nitinol tube and then heat set and electropolish the resultant structure to create a framework of struts and connecting elements. This framework can be any of huge range of shapes as disclosed herein and may be rendered visible under fluoroscopy through the addition of alloying elements (e.g., Platinum) or through a variety of other coatings or marker bands.

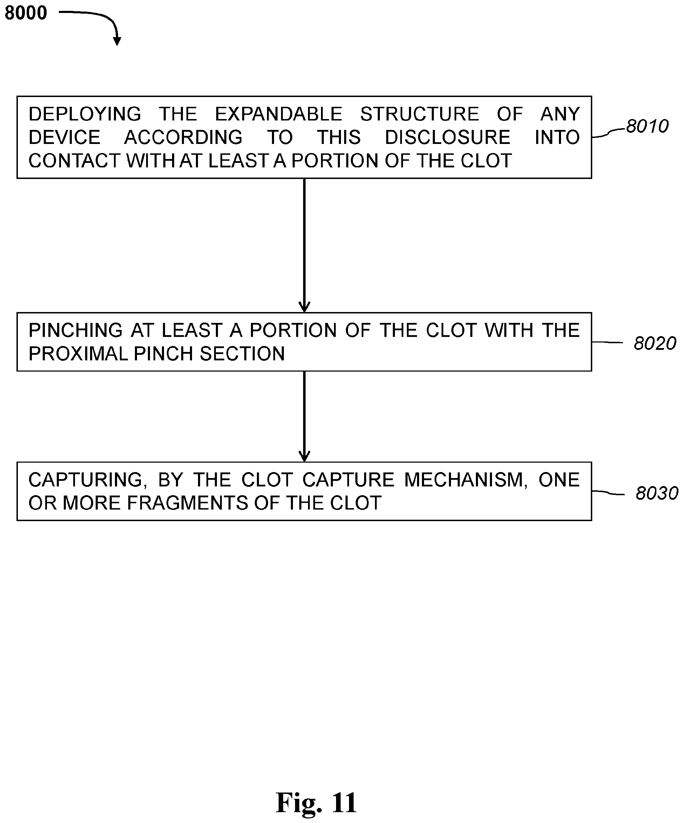

Fig. 11 is a flow diagram illustrating a method of removing a clot from a blood vessel of a patient, according to aspects of the present disclosure. The method steps in Fig. 11 can be implemented by any of the example means described herein or by similar means, as will be appreciated. Referring to method 8000 as outlined in Fig. 11, in step 1010, deploying an expandable structure of a device described in this disclosure into contact with at least a portion of the clot. In step 8020, method 8000 includes pinching at least a portion of the clot with the proximal pinch section. In step 8030, method 8000 includes capturing, by the clot capture mechanism, one or more fragments of the clot

The disclosure is not limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near to or a direction towards the physician.

In describing examples, terminology is resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method can be performed in a different order than those described herein without departing from the scope of the disclosed technology. Similarly, it is also to be understood that the mention of one or more components in a device or system does not preclude the presence of additional components or intervening components between those components expressly identified.

As discussed herein, a "patient" or "subject" can be a human or any animal. It should be appreciated that an animal can be a variety of any applicable type, including, but not limited to, mammal, veterinarian animal, livestock animal or pet-type animal, etc. As an example, the animal can be a laboratory animal specifically selected to have certain characteristics similar to a human (e.g., rat, dog, pig, monkey, or the like).

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

By "comprising" or "containing" or "including" is meant that at least the named compound, element, particle, or method step is present in the composition or article or method, but does not exclude the presence of other compounds, materials, particles, method steps, even if the other such compounds, material, particles, method steps have the same function as what is named.

It must also be noted that, as used in the specification and the appended claims, the singular forms "a," "an," and "the" include plural referents unless the context clearly dictates otherwise. Ranges can be expressed herein as from "about" or "approximately" one particular value and/or to "about" or "approximately" another particular value. When such a range is expressed, other exemplary embodiments include from the one particular value and/or to the other particular value.

The descriptions contained herein are examples of the disclosure and are not intended in any way to limit the scope of the disclosure. While particular examples of the present disclosure are described, various modifications to devices and methods can be made without departing from the scope and spirit of the disclosure. For example, while the examples described herein refer to particular components, the disclosure includes other examples utilizing various combinations of components to achieve a described functionality, utilizing alternative materials to achieve a described functionality, combining components from the various examples, combining components from the various example with known components, etc. The disclosure contemplates substitutions of component parts illustrated herein with other well-known and commercially-available products. To those having ordinary skill in the art to which this disclosure relates, these modifications are often apparent and are intended to be within the scope of the claims which follow.

## Claims

1. A clot removal device for removing clot from a vessel, comprising:
an expandable structure comprising a plurality of interconnected struts configured to engage with clot and comprising a constrained delivery configuration, an expanded clot engaging deployed configuration, the interconnected struts being configured in the expanded clot engaging deployed configuration so at least a portion of the plurality of interconnected struts interpenetrates clot, the expandable structure comprising:
a distal section; and
a proximal pinch section proximal of the distal section, the proximal pinch section configured to pinch clot on movement from the expanded clot engaging deployed configuration to an at least partially constrained clot pinching configuration; and
a clot capture mechanism comprising:
a proximal end,
a distal end,
a shaft extended from the proximal end and distally extended from a proximal end of the proximal pinch section; and
a capture member extended from the distal end of the shaft and within the distal section.

2. The device of Claim 1, the shaft comprising a curve and/or pitch equivalent to the pinch section.

3. The device of Claim 1, the expandable structure comprising an open distal end.

4. The device of Claim 1, the capture member further comprising a proximal mouth structure comprising a diameter approximately equal to a diameter of the distal section of the expandable structure, the proximal mouth structure comprising a fragment protection member with closed cells smaller than closed cells of the distal and proximal pinch sections, a distalmost end of the proximal mouth structure being positioned at the distal end of the distal section.

5. The device of Claim 1, a proximal end of the capture member positioned at or adjacent where the proximal pinch section meets the distal section.

6. The device of Claim 1, the distal section of the expandable structure further comprising a distal barrel section flared outwardly to form a barrel shape with an open distal end, and the interconnected struts comprise at least a plurality of proximally facing struts and a plurality of distally facing struts,
a length of at least some of the proximally facing struts being larger than a length of at least some of the distally facing struts thereby varying an outward radial force of the device in the expanded clot engaging deployed configuration.

7. The device of Claim 1, wherein the shaft of the clot capture mechanism and the proximal pinch section comprises a spiral form comprising a spiral pitch ranging between approximately 10-25 mm.

8. The device of Claim 1, further comprising: a longitudinal axis extended through the distal section, the shaft of the clot capture mechanism and the proximal pinch section extending around the longitudinal axis in a spiral.

9. The device of Claim 1, the proximal pinch section further comprising a clot engaging element configured to exert an outward radial force when deployed within the blood vessel having an inner diameter which is lower than that of the expanded deployed configuration, the clot engaging element being configured to exert an outward radial force varying in a generally sinusoidal pattern along a length of the clot engaging element.

10. The device of Claim 1, the distal section comprising:
an inner expandable body comprising a framework of struts; and
an outer expandable body comprising a framework of struts forming an outer body radially surrounding the inner body during the constrained delivery configuration and the expanded deployed configuration, wherein the outer expandable body is expandable to a radial extent to define a clot reception space.

11. The device of Claim 10, the framework of struts of the outer expandable body comprising a plurality of discontinuous expandable members spaced from adjacent expandable members, struts of each expandable forming closed cells with at least some struts terminating in radially separated distal apexes free from connection to an adjacent closed cell.

12. The device of Claim 1, the proximal pinch section comprising a diameter more than a diameter of the distal section, wherein proximal struts of the expandable structure (i) pass through one or more cells of the proximal pinch section, or (ii) are positioned both inside and outside of the proximal pinch section .

13. The device of Claim 1, the proximal pinch section comprising a diameter more than a diameter of the distal section, the distal section comprising an inner expandable body comprising a porous inner body flow channel.

14. The device of Claim 13, the capture member comprising:
an outer expandable body comprising a framework of struts forming an outer body radially surrounding the distal section during the constrained delivery configuration and the expanded deployed configuration, wherein the outer expandable body is expandable to a radial extent to define a clot reception space between it and the distal section .

15. The device of Claim 14, the outer expandable body comprising a closed distal end and at least one scaffolding segment proximal of the closed distal end, the at least one scaffolding segment comprising a plurality of closed cells, the distal section extending inside the least one scaffolding segment.

16. The device of Claim 15, the closed end formed by a distal scaffolding zone, comprising a plurality of struts that distally taper with closed cells smaller than cells proximal thereof in the outer expandable body .

17. The device of Claim 16, the distal section comprising an inner expandable body comprising a porous inner body flow channel terminating in a plurality of distal struts each having a first end coupled to a distal end of the porous inner body flow channel and a second end coupled to each other so as to form a connection point.

18. The device of Claim 17, the plurality of distal struts being spiraled, or configured in a bulged or flared pattern.
